# FASCICULE DE BREVET EUROPEEN

(11) **EP 0 811 353 B1**
(45) Date de publication et mention de la délivrance du brevet: **10.09.2003**
(21) Numéro de dépôt: 97450011.8
(22) Date de dépôt: 03.06.1997
(51) Int. Cl.: A61B 5/117, A43D 1/02

(54) **Procédé de prises d'empreintes, notamment pour la réalisation de chaussures orthopédiques et matériau adapté**
Verfahren zur Aufnahme von Abdrücken, insbesondere zur Herstellung von orthopädischen Schuhen und entsprechendem Material
Process for taking prints, particularly for manufacturing orthopaedic shoes and applied material

(30) Priorité: 04.06.1996 FR 9607168
(43) Date de publication de la demande: 10.12.1997
(73) Titulaire: Société des Etablissements Hilaire Gabilly Orthopédie Podologie Niort S.A., 79000 Niort (FR)
(72) Inventeur: Gabilly, Daniel, 79000 Niort (FR)
(74) Mandataire: Thébault, Jean-Louis

(56) Documents cités:
- EP-A- 0 323 099
- WO-A-90/05504
- WO-A-93/21967
- DE-A- 4 224 827

## Description

La présente invention concerne un procédé de prises d'empreintes pour la réalisation de produits adaptés à la morphologie d'une partie de l'anatomie d'une personne, notamment en vue de la réalisation de chaussures d'orthopédie. L'invention concerne aussi le matériau permettant la mise en oeuvre d'un tel procédé.

Les podo-orthésistes par exemple, ont en charge la réalisation de chaussures permettant à des personnes ayant des pieds déformés ou des atrophies ou encore des mutilations, de se chausser pour la marche.

La première étape consiste à prendre l'empreinte du pied de la personne. Cette étape est réalisée actuellement à l'aide de bandes de plâtre. Ces bandes sont enroulées autour du pied après avoir été humidifiées pour assurer l'activité du plâtre et qu'il puisse prendre.

On remarque les contraintes liées à la mise en place de ces bandes, tant du point de vue de la manipulation qui requiert un certain tour de main que du point de vue du confort de la personne dont le pied et les habits doivent être protégés d'éventuelles éclaboussures de plâtre.

Même si les temps de prise ont été réduits, ils ne peuvent pas l'être trop car la mise en place des bandes nécessite un certain temps et il n'est pas possible de retarder la prise en cours de mise en place, ce qui explique une marge de sécurité qui augmente sensiblement le temps de prise d'une empreinte en plâtre.

Cette empreinte est une réplique femelle et il convient de réaliser l'empreinte mâle pour la réalisation et les essais premiers de la chaussure.

La bande une fois le temps de séchage écoulé est découpée pour être retirée du pied de la personne.

On coule ensuite du polyuréthanne pour obtenir une forme positive et cette forme brute est reprise par usinage et rectifiée. La forme rectifiée et donc ayant un état de surface tout à fait adapté permet au technicien de réaliser la chaussure.

Cette méthode est d'un prix de revient en matériau qui est peu élevé, mais le temps de main d'oeuvre est plus important et elle procure aussi des désagréments qui ont été énoncés ci-avant.

Il serait intéressant de pouvoir disposer d'un procédé propre utilisant un matériau qui assure la prise d'empreinte de façon fine et qui permette de renouveler ces prises plusieurs fois.

Le but serait de pouvoir ajuster au mieux la conformation du matériau au pied de la personne et donc de pouvoir renouveler la prise quasi-instantanément si cela est nécessaire.

Le but de la présente invention est de proposer un procédé de prises d'empreintes d'une partie de l'anatomie d'un être humain, notamment d'un pied pour la réalisation de chaussures en orthopédie, qui se caractérise en ce qu'il comprend les étapes suivantes :
- réalisation d'une préforme d'un profil simple donné en matériau polymère à mémoire de forme, sensiblement rigide à température ambiante et souple et élastique à sa température de travail, dans tous les cas une température compatible avec l'épiderme humain,
- mise en température de travail de la préforme,
- plaquage de la préforme sur la partie concernée,
- attente du refroidissement de la préforme en dessous de sa température de travail,
- retrait de la préforme de la partie concernée et coulage de la forme positive, et
- remise de la préforme au profil simple initial par montée de nouveau à la température de travail sans contrainte, afin de pouvoir l'utiliser de nouveau pour une autre prise d'empreinte.

Plus particulièrement, on porte la préforme à la température de travail par passage dans un bain d'eau chaude ou dans une étuve à air chaud.

L'invention concerne aussi la réalisation de la préforme qui comprend les étapes suivantes :
- trempage dans un bain ou pulvérisation d'un gabarit de profil simple avec une composition de polymères à mémoire de forme diluée dans un solvant,
- évaporation du solvant, et
- découpe de la préforme en polymère suivant des lignes adaptées pour permettre le retrait du gabarit, et
- mise en place de moyens de fermeture rapide.

Une composition de polymère pour la réalisation d'une préforme en vue de la mise en oeuvre du procédé comprend au moins un élastomère et une polycaprolactone dilués dans une composition de solvants adaptée pour solubiliser au moins l'un et l'autre des polymères.

Plus particulièrement, l'élastomère est du polyuréthanne.

Quant à la composition de solvants, elle comprend du diméthylformamide pour solubiliser le polyuréthanne, du toluène pour solubiliser la polycaprolactone et du méthyléthylcétone comme tampon.

L'invention est décrite ci-après selon un mode de réalisation particulier, non limitatif.

Il convient tout d'abord de réaliser une préforme en un matériau polymère ayant une mémoire de forme. Ces préformes sont élaborées à partir de gabarits, éventuellement spécifiques sans qu'il soit nécessaire d'engager des sommes importantes pour la fabrication de ces gabarits. Ces gabarits sont réalisés généralement en fonction des gradations connues pour permettre une adaptation à chacune des pointures ou une série de pointures de la gamme d'un chausseur non orthopédiste mais au-delà souvent pour tenir compte des cas extrêmes. On peut aussi prévoir une gamme de préformes suivant un étalonnage particulier.

Ces préformes sont réalisées par trempage des gabarits disponibles dans des bains de polymère à mémoire de forme dilué dans un solvant ou par pulvérisation de ce polymère à mémoire de forme dont la dilution est adaptée.

Une fois le solvant évaporé, la coquille en polymère est découpée de façon adaptée pour en assurer le retrait.

Au droit de ces découpes il convient d'intégrer des moyens de fermeture tels que des bandes à accrochage rapide. Cette intégration peut être réalisée postérieurement mais avantageusement, elle peut l'être au moment de la pulvérisation par exemple.

On obtient ainsi une préforme ayant un profil sensiblement standard, avec des proportions variant en fonction des pointures ceci de façon connue.

Lorsque le podo-orthésiste souhaite faire une prise d'empreinte du pied d'une personne, il choisit parmi ses préformes, celle qui se rapproche le plus du pied concerné et met en oeuvre une autre étape du procédé selon l'invention.

Il porte la préforme à une température adaptée en vue de la rendre apte à la déformation. Il ouvre les moyens de fermeture pour permettre l'enfilage de la préforme comme une chaussette sur le pied et les moyens de fermeture sont mis en place.

La température de la préforme est nécessairement tout à fait supportable par l'épiderme. Par exemple on peut imaginer de chauffer la préforme uniquement en la trempant dans un bain à 60°C ou mieux encore dans une étuve à air chaud thermostatée qui évite la manipulation d'eau même si celle-ci ne risque pas d'engendrer des risques de tâches ou de dégâts dans les lieux de prises d'empreintes comme aurait pu en provoquer le plâtre.

La préforme ainsi enfilée, est déformée manuellement si nécessaire, en plus de son élasticité propre pour s'assurer qu'elle épouse bien le contour du pied et notamment la partie du pied qui pose problème.

Très rapidement, le refroidissement provoque une rigidification suffisante de cette préforme et il est possible de procéder au retrait de cette préforme par ouverture des moyens de fermeture.

Si la prise n'est pas satisfaisante, on porte de nouveau la même préforme en température puis on renouvelle l'opération ce qui ne demande que quelques secondes.

Si la prise est satisfaisante, on procède à la fabrication de la forme positive en polyuréthanne mais dans ce cas, l'usinage et la rectification sont inutiles car l'état de surface de la préforme est tel qu'il donne une forme positive directement utilisable pour la fabrication de la chaussure.

La réaction de polymérisation du polyuréthanne est faiblement exothermique si bien que la préforme conserve intégralement l'empreinte.

La préforme, une fois la chaussure réalisée, est ramenée à son profil initial par montée en température. En l'absence de déformation forcée par introduction d'un élément à l'intérieur, la préforme reprend le profil qu'elle avait après évaporation des solvants, c'est à dire celle du gabarit.

Plus particulièrement, un matériau utilisable pour la mise en oeuvre de ce procédé est une composition d'un élastomère et d'une polycaprolactone.

En effet, l'élastomère assure l'effet élastique pour épouser les contours et la polycaprolactone l'effet de rigidification et de blocage des capacités de l'élastomère à température ambiante avec un ramollissement suffisant pour une faible élévation de température.

Un matériau pouvant donner satisfaction pour la réalisation de telles préformes comprend les deux polymères dans une composition de solvants.

Le premier polymère est du polyuréthanne et le second polymère est de la polycaprolactone.

La composition de solvants comprend trois solvants le diméthylformamide, la méthyléthylcétone et le toluène respectivement comme solvant du polyuréthanne, tampon et solvant de la polycaprolactone ,

Il faut en effet éviter le bullage lors de l'évaporation du solvant, la ségrégation et autres effets parasites tout en assurant une évaporation rapide des solvants.

Selon un perfectionnement de l'invention, on peut intégrer dans la préforme des témoins de température par des pigments sensibles à la chaleur et dont la couleur varie en fonction de la température si bien que pour une couleur donnée, on sait que la température de la préforme est à la température de ramollissement suffisante pour la prise d'empreinte ou au contraire que la préforme est à la température de rigidification permettant son retrait.

On peut aussi envisager de disposer des éléments d'information comme les pointures par exemple dans l'épaisseur de la préforme mais aussi des éléments germicides et fongicide pour assurer un traitement permanent de la préforme.

Selon d'autres perfectionnements de l'invention, on peut aussi prévoir une couche supplémentaire interne pour éviter à la préforme de coller de façon trop importante sur la peau lors de l'enfilage.

Un traitement par rayonnement peut aussi permettre la diminution de cette propriété de coller tout en augmentant les capacités élastiques de la préforme lorsqu'elle est portée en température.

Toute la description a été menée en regard d'un produit orthopédique afin de donner tous les détails du procédé selon l'invention mais cet exemple de réalisation ne doit pas être considéré comme limitatif.

En effet, un tel matériau est en lui-même susceptible de trouver des applications importantes dans le milieu médical comme moyen de contention post-traumatique ou chirurgical ou encore comme orthèse de compression pour brûlés.

En rééducation, on peut réaliser des attelles adaptables.

## Revendications

1. Procédé de prises d'empreintes d'une partie de l'anatomie d'un être humain, notamment d'un pied pour la réalisation de chaussures en orthopédie ou d'un moyen de contention, **caractérisé en ce qu'**il comprend les étapes suivantes :
- réalisation d'une préforme d'un profil simple donné en matériau polymère à mémoire de forme, sensiblement rigide à température ambiante et souple et élastique à sa température de travail,
- mise en température de travail de la préforme,
- plaquage de la préforme sur la partie concernée,
- attente du refroidissement de la préforme en dessous de sa température de travail,
- retrait de la préforme de la partie concernée et coulage de la forme positive, et
- remise de la préforme au profil simple initial par montée à nouveau à la température de travail sans contrainte.

2. Procédé selon la revendication 1, **caractérisé en ce que** l'on porte la préforme à la température de travail par passage dans un bain d'eau chaude.

3. Procédé selon la revendication 1, **caractérisé en ce que** l'on porte la préforme à la température de travail par passage dans une étuve à air chaud.

4. Procédé de fabrication d'une préforme pour la mise en oeuvre du procédé selon l'une quelconque des revendications 1 à 3, **caractérisé par** les étapes suivantes
- trempage ou pulvérisation d'un gabarit de profil simple dans un bain comprenant une composition de polymères à mémoire de forme diluée dans un solvant,
- séchage du solvant,
- découpe de la préforme en polymère suivant des lignes adaptées pour en permettre le retrait du gabarit, et
- mise en place de moyens de fermeture rapide.

5. Procédé selon la revendication 4 **caractérisé en ce que** la dite composition de polymères comprend au moins un élastomère et une polycaprolactone dilués dans une composition de solvants adaptés pour solubiliser au moins l'un et l'autre des polymères.

6. Procédé selon la revendication 5, **caractérisé en ce que** l'élastomère est du polyuréthanne.

7. Procédé selon la revendication 5 ou 6, **caractérisé en ce que** la composition de solvants comprend du diméthylformamide pour solubiliser le polyuréthanne, du toluène pour solubiliser la polycaprolactone et de la méthyléthylcétone comme tampon.

## Patentansprüche

1. Verfahren zum Nehmen eines Abdrucks von einem Teil der Anatomie eines menschlichen Wesens, insbesondere von einem Fuß, für die Herstellung von orthopädischen Schuhen oder eines Fixierungsmittels, **dadurch gekennzeichnet, daß** es die folgenden Schritte umfaßt:
- Herstellen eines Vorformlings mit einem gegebenen einfachen Profil aus einem Polymerwerkstoff mit Formerinnerungsvermögen, der bei Umgebungstemperatur im wesentlichen starr und bei seiner Arbeitstemperatur weich und elastisch ist,
- Bringen des Vorformlings auf die Arbeitstemperatur,
- Aufbringen des Vorformlings auf den betreffenden Teil,
- Abwarten, bis der Vorformling unter seine Arbeitstemperatur abgekühlt ist,
- Abnehmen des Vorformlings von dem betreffenden Teil und Gießen der positiven Form und
- Zurückführen des Vorformlings ohne Zwang zu dem anfänglichen einfachen Profil durch erneute Erwärmung auf die Arbeitstemperatur.

2. Verfahren nach Anspruch 1, **dadurch gekennzeichnet, daß** der Vorformling durch Bewegen in einem Heißwasserbad auf der Arbeitstemperatur gehalten wird.

3. Verfahren nach Anspruch 1, **dadurch gekennzeichnet, daß** der Vorformling durch Bewegen in einem Heißluftschrank auf der Arbeitstemperatur gehalten wird.

4. Verfahren für die Herstellung eines Vorformlings für die Ausführung des Verfahrens nach einem der Ansprüche 1. bis 3, **gekennzeichnet durch** die folgenden Schritte:
- Tauchbeschichten oder Pulverbeschichten einer Schablone mit einfachem Profil in einem Bad, das eine Polymerzusammensetzung mit Formerinnerungsvermögen, die in einem Lösungsmittel aufgelöst ist, umfaßt,
- Trocknen des Lösungsmittels,
- Schneiden des Polymervorformlings längs geeigneter Linien, um die Schablone entnehmen zu können, und
- Anordnen von Schnellverschlußmitteln:

5. Verfahren nach Anspruch 4, **dadurch gekennzeichnet, daß** die Polymerverbindung wenigstens ein Elastomer und ein Polycaprolacton, die in einer Zusammensetzung von Lösungsmitteln aufgelöst sind, die wenigstens das eine oder das andere der Polymere auflösen können, umfaßt.

6. Verfahren nach Anspruch 5, **dadurch gekennzeichnet, daß** das Elastomer Polyurethan ist.

7. Verfahren nach Anspruch 5 oder 6, **dadurch gekennzeichnet, daß** die Zusammensetzung von Lösungsmitteln Dimethylformamid umfaßt, um Polyurethan aufzulösen, Toluol umfaßt, um Polycaprolacton aufzulösen, und Methylethylketon als Puffer umfaßt.

## Claims

1. A method of taking impressions of part of the anatomy of a human being, in particular of a foot for producing orthopaedic footwear or a restraint means, **characterised in that** it comprises the following steps:
- producing a preform with a given simple profile made from polymer material with a shape memory, substantially rigid at room temperature and flexible and elastic at its working temperature,
- raising the preform to working temperature,
- pressing the preform on the part concerned,
- awaiting the cooling of the preform below its working temperature,
- removing the preform from the part concerned and casting the positive shape, and
- returning the preform to the initial simple profile by once again raising it to the working temperature without stress.

2. A method according to Claim 1, **characterised in that** the preform is raised to the working temperature by passing through a hot-water bath.

3. A method according to Claim 1, **characterised in that** the preform is raised to the working temperature by passing through a hot-air oven.

4. A method of manufacturing a preform for implementing the method according to any one of Claims 1 to 3, **characterised by** the following steps:
- soaking or spraying of a simple profile template in a bath comprising a composition of polymers with shape memory diluted in a solvent,
- drying the solvent,
- cutting the polymer preform along lines adapted to enable the template to be removed from it, and
- fitting quick closure means.

5. A method according to Claim 4, **characterised in that** the polymer composition comprises at least one elastomer and one polycaprolactone diluted in a composition of solvents adapted to solubilise at least both of the polymers.

6. A method according to Claim 5, **characterised in that** the elastomer is polyurethane.

7. A method according to Claim 5 or 6, **characterised in that** the solvent composition comprises dimethylformamide for solubilising the polyurethane, toluene for solubilising the polycaprolactone and methyl ethyl ketone as a buffer.
